# EUROPEAN PATENT APPLICATION

(11) **EP 1 462 954 A2**
(43) Date of publication of application: **29.09.2004**
(21) Application number: 04002926.6
(22) Date of filing: 10.02.2004
(51) Int. Cl.: G06F 17/30

(54) **Key word frequency calculation method and program for carrying out the same**

(30) Priority: 28.03.2003 JP 2003092098
(71) Applicant: Hitachi Software Engineering Co., Ltd., Yokohama-shi, Kanagawa 230-0045 (JP)
(72) Inventor: Tago, Shigeru, Hitachi Software Eng. Co., Ltd., Tokyo 140-0002 (JP); Yoshii, Junji, Hitachi Software Eng. Co., Ltd., Tokyo 140-0002 (JP); Mizunuma, Tadashi, Hitachi Software Eng. Co., Ltd., Tokyo 140-0002 (JP)
(74) Representative: Liesegang, Roland, Dr.-Ing.

(57) **Abstract**

The frequency of appearance of a keyword is calculated using a first database in which information about a base sequence and an amino acid sequence are stored, and a second database in which text data is stored. A keyword frequency calculation method includes a first text data extraction step for extracting first text data from said first database based on a base sequence or an amino acid sequence inputted by a user; an identifier extraction step for extracting an identifier identifying text data in said first text data from said first text data; a second text data extraction step for extracting second text data from said second database based on said identifier; and an appearance frequency calculation step for sequentially reading keywords from a keyword table containing keywords related to said first database, and for calculating the frequency of appearance of each of said keywords in said second text data.

## Description

### BACKGROUND OF THE INVENTION

### Field of the Invention

The present invention relates to a database search technique suitable for the retrieval of gene-related data. Particularly, the invention relates to a database search technique for detecting the frequency of a keyword contained in document data, using a text mining method.

### Background Art

Generally, there are two kinds of databases for document data describing results of research into genes or proteins. A first database describes the base sequences or amino acid sequences that are the themes of study. A second database describes the functions or characteristics of genes or proteins that have the aforementioned sequences. The data in the first database usually describes, together with the base or amino acid sequence information, an identifier in the form of related text data for document data in the second database that describes the same gene or protein.

Searchers seeking the function or characteristics of a particular gene or protein have been so far provided with any of the following methods. In one method, the aforementioned first database is searched using the sequence information of the gene or protein as a search key. An identifier for data in the second database is extracted from the data obtained from the first database, and then the data in the second database is obtained. Referring to that data, the searcher can then learn the function or characteristics of the gene or protein described therein. As an example of this method, a method called BLAST (http://www.ncbi.nlm.nih.gov/BLAST/) is widely employed.

In a second method, an identifier of a particular gene or protein, or related information of a similar kind, is selected as one or more keywords different from the sequence information. Data is extracted from the second database that contains any of the keywords, and the searcher can then refer to that data to understand the function or characteristics of the gene or protein described therein. A method of narrowing the number of items of data extracted from the second database, utilizing information corresponding to knowledge, is disclosed in JP Patent Publication (Kokai) No. 2002-32374 entitled "Information extraction method and recording medium."
Patent Document 1: JP Patent Publication (Kokai) No. 2002-32374

### SUMMARY OF THE INVENTION

The above-described conventional methods have the following problems. Namely, in the first method, the searcher must refer to the data in the second database directly and therefore must refer to a great quantity of document data in order to figure out the function or characteristics of a particular gene or protein.

In the second method, while it is possible to extract an appropriate document data group as long as an appropriate keyword can be selected, selecting an appropriate keyword is difficult for a searcher with no knowledge about what kind of function or characteristics the gene or protein with a particular base or amino acid sequence might possess. Actually, it is those who wish to know the function or characteristics of a particular gene or protein that conduct the search, and so the difficulty with which the searcher must select an appropriate keyword is obvious. Thus, it has been difficult to extract an appropriate document data group.

The invention provides a method of calculating the frequency of appearance of a keyword, using a first database in which information about a base sequence or an amino acid sequence is stored and a second database in which document data is stored, said method comprising: a first text data extraction step for extracting first text data from said first database based on a base sequence or an amino acid sequence inputted by a user; an identifier extraction step for extracting an identifier identifying document data in said first text data from said first text data; a second text data extraction step for extracting second text data from said second database based on said identifier; and an appearance frequency calculation step for sequentially reading keywords from a keyword table containing keywords related to said first database, and for calculating the frequency of appearance of each of said keywords in said second text data.

In accordance with the invention, when a searcher wishes to know the function or characteristics of a gene or protein with a particular sequence, the searcher can be provided with a list of keywords indicating the function or characteristics of the gene or protein by entering the sequence information itself as a search key, the list showing the keywords in terms of the importance, or the frequency of appearance in document data.

Further, by entering a plurality of sequences as search keys, a list of keywords indicating the functions or characteristics common to a plurality of genes or proteins can be obtained.

### BRIEF DESCRIPTION OF THE DRAWINGS

Fig. 1 shows the configuration of a database search system according to the invention.
Fig. 2 shows the structure of a first text data file.
Fig. 3 shows the structure of a second text data file.
Fig. 4 shows an example of a sequence character string input page.
Fig. 5 shows the structure of a category table.
Fig. 6 shows the structure of a frequency calculation result table.
Fig. 7 shows the structure of a frequency table of a tree structure.
Fig. 8 shows the flow of the operation of the database search system according to the invention.

### DESCRIPTION OF THE PREFERRED EMBODIMENTS

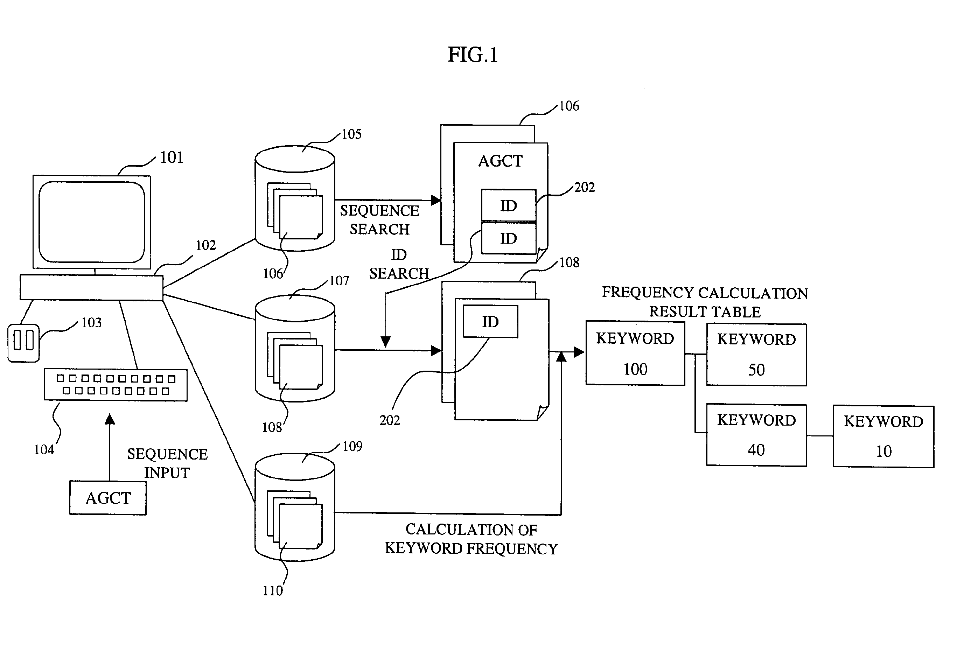

The invention will now be described by way of a preferred embodiment thereof with reference made to the drawings. Fig. 1 shows the configuration of a system for database search according to the present invention. The database search system includes a display unit 101, a calculating unit 102, a mouse unit 103, a keyboard 104, and a first, second and third file systems 105, 107 and 109.

The display unit 101 has the functions of displaying characters, figures and a mouse cursor. The calculating unit 102 has the functions of receiving the position of the mouse cursor on the display unit 101, receiving an arbitrary character string from the keyboard, retaining data in a memory, cutting out a particular portion of text data, and determining whether or not particular character strings correspond with each other. The mouse unit 103 has the functions of instructing the movement of the mouse cursor on the display unit 101, and instructing the recognition of the position of the mouse cursor upon the pressing of a button. The keyboard 104 has the function of entering an arbitrary character string and sending it to the calculating unit 102.

A first file system 105 is an auxiliary storage unit with the function of retaining text data 106 in individual files. A second file system 107 is an auxiliary storage unit with the function of retaining text data 108 in individual files. A third file system 109 is an auxiliary storage unit with the function of retaining a category table 110 in files.

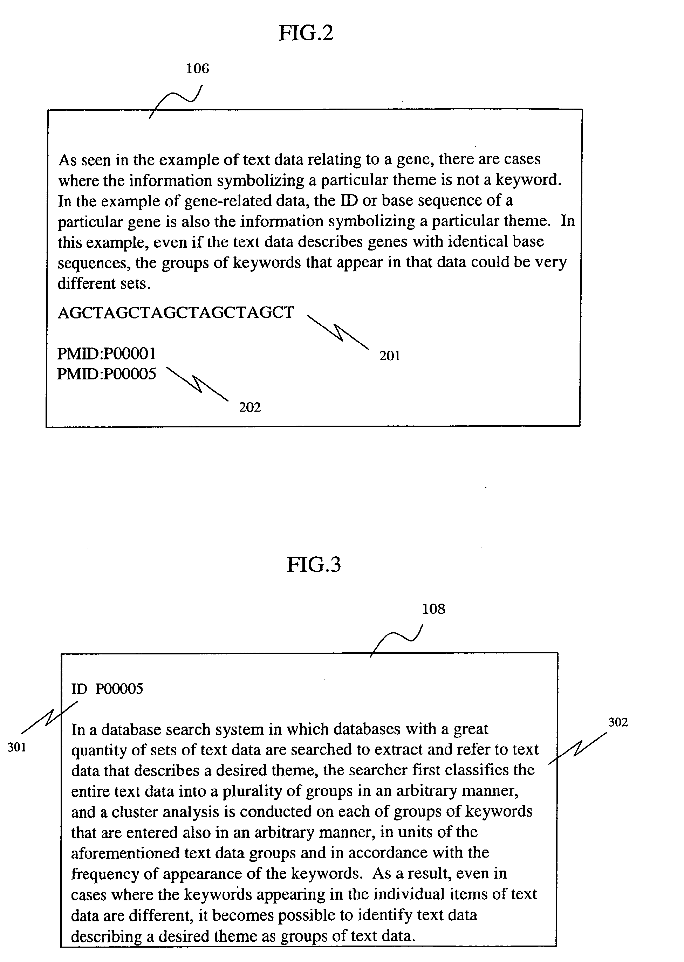

Fig. 2 shows the structure of the text data 106 in the first file system 105. In this example, the data is in the form of a thesis describing the result of research into a particular base sequence. The text data 106 includes a base or amino acid sequence 201 as the subject of description in the data, and an identifier 202 of other text data in which there is description related to the present data. In the illustrated example, there are two items of related text data with respect to the present data, two identifiers are stored. In this example, the identifiers are indicated as PMID (PubMed ID).

Fig. 3 shows the structure of the text data 108 in the second file system 107. The text data 108 includes an identifier 301 of the present data, and a character string 302 corresponding to the main text of the present data. In the illustrated example, the data describes the result of molecular-biological study into a gene or protein, for example.

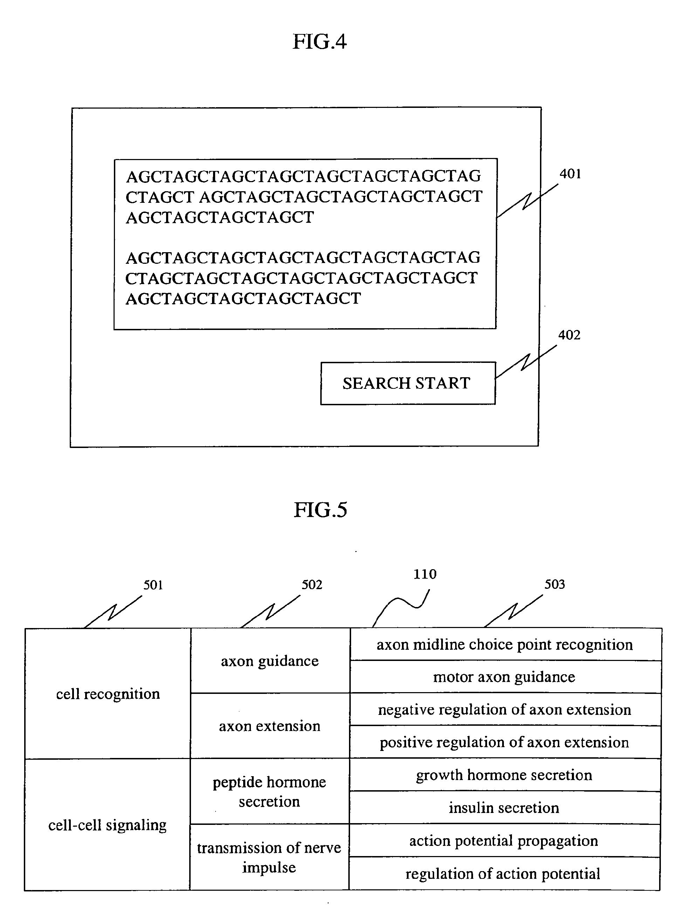

Fig. 4 shows a search start page displayed on the display unit 101. The search start page includes a field 401 for the input of the sequence of a base or amino acid in the form of a character string, and a search start button 402 for instructing the calculating unit 102 to start a search, both of which are operated by the user.

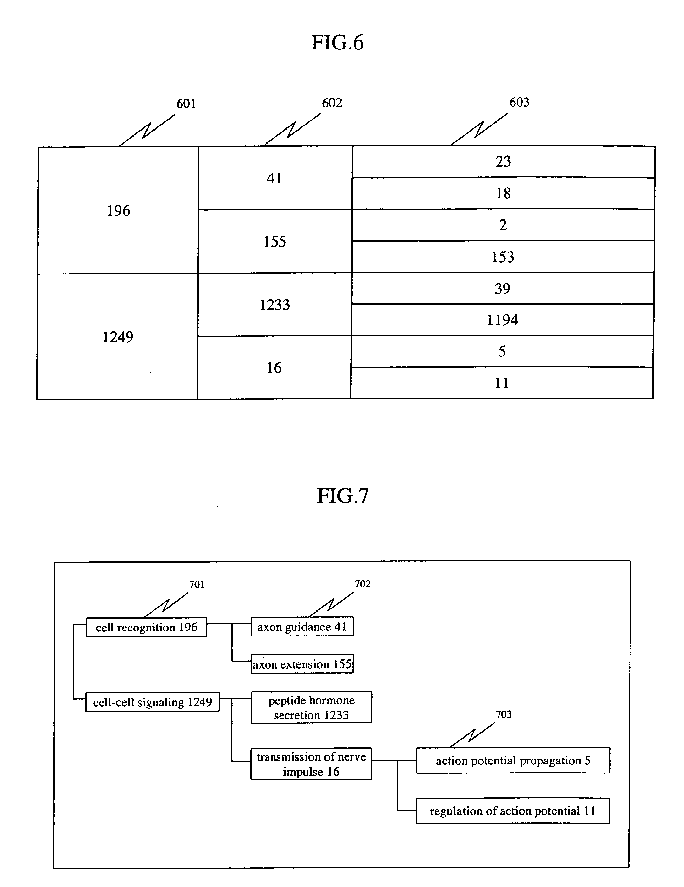

Fig. 5 shows the structure of the category table 110 in the third file system 109. The category table 110 includes a category portion 501 for the storage of the name of a category to which one or more keywords belong, a lower category portion 502 for the storage of the names of lower-level categories, and a keyword portion 503 for the storage of keywords. The keywords contained in the category table 110 may include only those keywords that are related to the information contained the text data 108 in the second file system 107. In the illustrated example, it is indicated that lower-level categories "axon guidance" and "axon extension" belong to an upper-level category "cell recognition". It is also indicated that keyword "motor axon guidance" belongs to a lower-level category "axon guidance".

Referring back to Fig. 1, the concept of the database search system according to the invention will be described. A user enters a base or amino acid sequence, such as a base sequence AGCT, for example, using the keyboard 104. Based on the sequence AGCT, the calculating unit 102 extracts text data 106 from the first file system 105 that contains the sequence AGCT or information related thereto.

Each file of text data 106 contains identifier 202 for identifying document data. The calculating unit 102 extracts the identifier 202 from each file of text data 106, and extracts text data 108 from the second file system 107 which corresponds to the identifier 202.

The calculating unit 102 obtains keywords contained in the category table 110 in the third file system 109, and then calculates the frequency of appearance of the keywords in the extracted text data 108. Specifically, the number of files of extracted text data 108 in which each keyword appears or is used is calculated.

The user can thus learn the frequency of each keyword related to the sequence AGCT in the text data 108 in the second file system 107. In the category table 110, keywords are stored in a tree structure in which the keywords are classified according to category. Thus, the user can obtain a table on the screen of the display unit 101 showing the result of calculation of keyword frequencies in a tree structure.

Fig. 6 shows a frequency calculation result table showing the frequency of the keywords of Fig. 5 in the text data 108. As will be seen by comparing Figs. 5 and 6, in a region 601 of the frequency calculation result table, there is indicated the frequency of each category in the category portion 501 of the category table 110. In a region 602, there is indicated the frequency of each lower-level category in the lower-level category portion 502 of the category table 110. In a region 603, there is indicated the frequency of individual keywords in the keyword portion 503 of the category table 110.

The frequency of each category in the category portion 501 is the sum of the frequencies of the lower-level categories belonging to that category. The frequency of each lower-level category in the lower-category portion 502 is the sum of the frequencies of the keywords that belong to that lower-level category. Thus, the frequency of each and every category above the region 603 can be obtained by determining the frequencies of the keywords in the region 603.

In the illustrated example, the frequency of appearance of all of the keywords belonging to the category "cell recognition" is 196. This indicates that keywords belonging to the category "cell recognition" appear at least once in 196 files of the text data contained in the second file system 107.

The frequency of appearance of the keyword "motor axon guidance" is 18. This indicates that the total number of text data files in the second file system 107 in which the keyword "motor axon guidance" appears at least once is 18.

Fig. 7 shows a tree-structured table showing the results of calculation of the frequency of category and keyword, as displayed on the screen of the display unit 101. This table is generated by superposing the frequency calculation result table of Fig. 6 on the category table 110 of Fig. 5. Regions 701 and 702 in the tree-structured frequency table shown in Fig. 7 are graphic nodes corresponding to the category 501 and the lower-level category 502, respectively, in Fig. 5. A region 703 is a graphic node corresponding to the keyword 503 in Fig. 5.

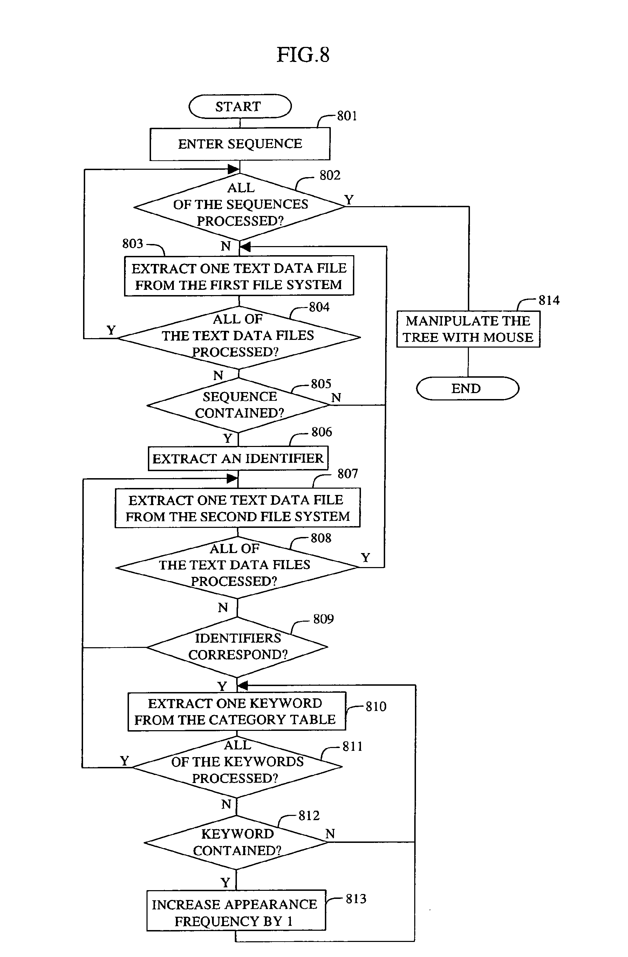

Now referring to Fig. 8, the flow of the procedure according to the database search method of the present invention will be described. In step 801, the user enters a character string representing a base or amino acid sequence in the input field 401 on the search start page of Fig. 4. In the example of Fig. 4, the sequence is expressed by arranging four bases A, G, C and T in a string. If a plurality of sequences are entered, a space is inserted between the character strings representing the individual sequences. The user then clicks the search start button 402 on the search start page of Fig. 4 using the mouse unit 103 to proceed to the next step 802.

In step 802, it is checked to see if all of the sequences entered in the input field 401 of the search start page of Fig. 4 have been processed. If all of the sequences have been processed, the routine proceeds to step 814, and if not, the routine proceeds to step 803.

In step 803, one text data file 106 is taken out from the first file system 105. In step 804, it is determined whether all of the text data files have been processed. If all of the text data files have been processed, the routine returns to step 802 where the next sequence is processed. If not, the routine proceeds to step 805, and the processes in step 803 and thereafter are repeated until it is determined in step 804 that all of the text data files have been processed.

In step 805, the sequence character string 201 is taken out from the text data file 106 obtained in step 803, and it is determined whether the sequence character string corresponds to, or contains part of, one of those sequence character strings entered in step 801 which is currently the subject of processing. The determination may be carried out using the aforementioned BLAST. If the sequence character string is contained, the routine proceeds to step 806. If not, the routine returns to step 803 where the next file is taken out and the subsequent steps are carried out.

Thereafter, in step 806, the identifier 202 is taken out from the text data file 106. In step 807, one of the text data files 108 is taken out from the second file system 107. In step 808, it is then determined whether all of the text data files in the second file system have been processed. If all of the text data files in the second file system have been processed, the routine returns to step 803 where the next file is taken out and the above-described processes are carried out. If not all of the text data files in the second file system have been processed, the subsequent steps are repeatedly carried out.

In step 809, the identifier 301 of the present data is taken out from the text data file 106, and it is then determined whether the identifier 301 corresponds to any of the identifiers 202 of text data files 106 taken out in step 806. If it does, the routine proceeds to step 810, and if not, the routine returns to step 807 where another file is taken out and the subsequent processes are carried out.

In step 810, one of the keywords is taken out from the category table 110. In step 811, it is then determined whether all of the keywords in the category table have been processed. If all of the keywords have been processed, the routine returns to step 807 and another file is processed. If not all of the keywords have been processed, the routine proceeds to step 812.

Thereafter, in step 812, it is examined to see if the keyword taken out in step 810 is contained in the text data file taken out in step 807. If not, the routine returns to step 810, where the next keyword is processed. If contained, the routine proceeds to step 813.

In step 813, the frequency value at that position in the keyword appearance frequency storage region 603 of the frequency calculation result table in Fig. 6 which corresponds to the keyword that has been processed is increased by one. At the same time, with regard to the categories 501 and 502 that are the upper-level categories for the keyword that has been processed, the frequency values at the corresponding positions in the keyword appearance frequency storage regions 601 and 602 are increased by one. The routine then returns to step 810.

Thus, if it is determined in step 802 that all of the sequence character strings have been processed, the routine proceeds to step 814.

In step 814, the tree-structured frequency table of Fig. 7 in which the contents of the category table of Fig. 5 and those of the frequency calculation result table of Fig. 6 are reflected is displayed on the display unit 101. By clicking a graphic node corresponding to any of the categories using the mouse unit, for example, a partial tree the user wishes to refer to can be displayed by switching, for example, between the display and non-display of the lower-level graphic nodes.

The processes in Fig. 8 may be carried out by a computer. Thus, the invention includes a program for causing a computer to carry out the processes of Fig. 8, and a recording medium in which such a program is stored.

While the invention has been described by way of an example thereof, the example is illustrative and not restrictive and it will be understood by those skilled in the art that various changes and modifications may be made in the invention without departing from the scope of the appended claims.

In accordance with the invention, when a searcher wishes to know the function or characteristics of a gene or protein with a particular sequence, the searcher can be provided with a list of keywords indicating the function or characteristics of the gene or protein by entering the sequence information itself as a search key, the list showing the keywords in terms of the importance, or the frequency of appearance in document data.

In accordance with the invention, by entering a plurality of sequences as search keys, a list of keywords indicating the functions or characteristics common to a plurality of genes or proteins can be obtained.

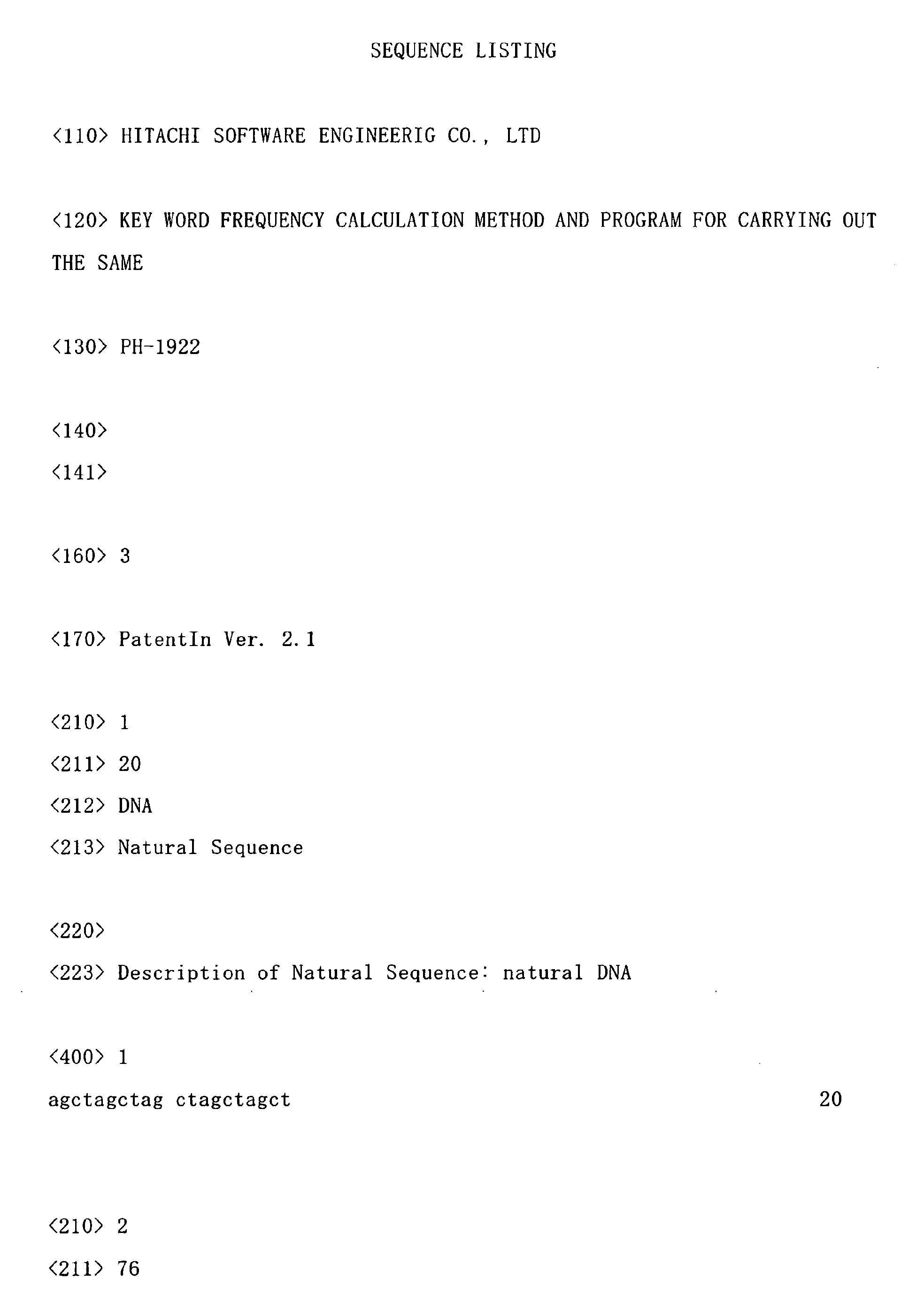

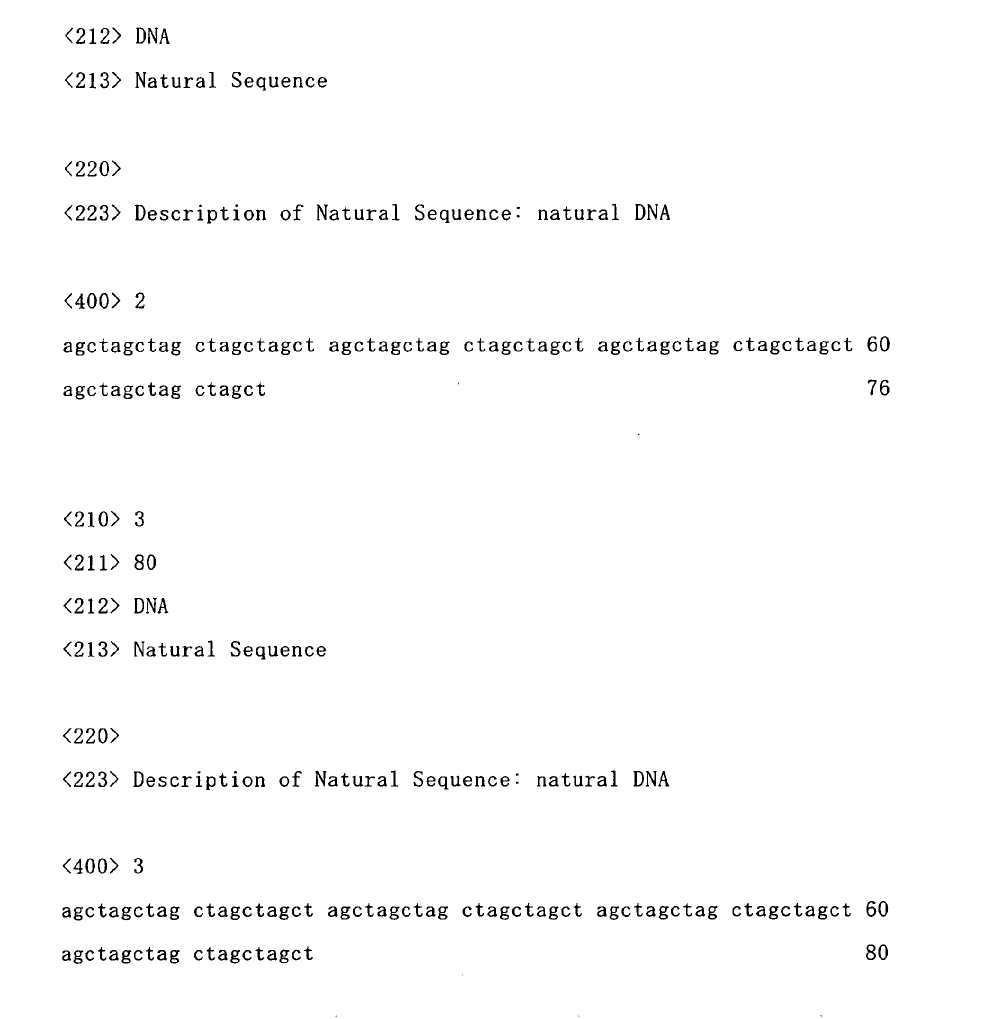

## Claims

1. A method of calculating the frequency of appearance of a keyword, using a first database in which information about a base sequence or an amino acid sequence is stored and a second database in which document data is stored, said method comprising:
a first text data extraction step for extracting first text data from said first database based on a base sequence or an amino acid sequence inputted by a user;
an identifier extraction step for extracting an identifier identifying document data in said first text data from said first text data;
a second text data extraction step for extracting second text data from said second database based on said identifier; and
an appearance frequency calculation step for sequentially reading keywords from a keyword table containing keywords related to said first database, and for calculating the frequency of appearance of each of said keywords in said second text data.

2. The keyword frequency calculating method according to claim 1, wherein said keyword table has a tree structure in which keywords are stored such that they are classified according to categories, and wherein said appearance frequency calculation step comprises a step for generating a frequency calculation result table of a tree structure, said table containing the frequency of appearance of a keyword and the frequency of appearance of an upper-level category to which the keyword belongs.

3. The keyword frequency calculating method according to claim 1.or 2, wherein said first text data extraction step comprises a step for extracting first text data from said first database for each of a plurality of sequences entered by the user.

4. A program for causing a computer to carry out the keyword frequency calculation method according to any one of claims 1 to 3.
